(19) **Europäisches Patentamt / European Patent Office / Office européen des brevets**

(11) **EP 2 979 757 A1**

(12) **EUROPEAN PATENT APPLICATION**
published in accordance with Art. 153(4) EPC

(43) Date of publication:
**03.02.2016 Bulletin 2016/05**

(21) Application number: **14775852.8**

(22) Date of filing: **24.03.2014**

(51) Int Cl.:
**B01J 27/199** (2006.01)   **B01J 37/00** (2006.01)
**B01J 37/04** (2006.01)   **B01J 37/08** (2006.01)
**C07C 51/235** (2006.01)   **C07C 57/055** (2006.01)
**C07B 61/00** (2006.01)

(86) International application number:
**PCT/JP2014/057990**

(87) International publication number:
**WO 2014/157040 (02.10.2014 Gazette 2014/40)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**

(30) Priority: **28.03.2013   JP 2013069908**

(71) Applicant: **Nippon Kayaku Kabushiki Kaisha Tokyo 100-0005 (JP)**

(72) Inventors:
• **SAKAI Hideomi**
  **Sanyoonoda-shi**
  **Yamaguchi 757-8686 (JP)**
• **KONNO Yosuke**
  **Sanyoonoda-shi**
  **Yamaguchi 757-8686 (JP)**
• **EJIRI Tomoyuki**
  **Sanyoonoda-shi**
  **Yamaguchi 757-8686 (JP)**
• **NISHIMURA Eiji**
  **Sanyoonoda-shi**
  **Yamaguchi 757-8686 (JP)**

(74) Representative: **Gille Hrabal**
  **Brucknerstrasse 20**
  **40593 Düsseldorf (DE)**

(54) **CATALYST FOR METHACRYLIC ACID PRODUCTION, METHOD FOR PRODUCING SAME, AND METHOD FOR PRODUCING METHACRYLIC ACID**

(57) Disclosed is a method for producing a catalyst for producing methacrylic acid by subjecting methacrolein or the like to vapor phase catalytic oxidation, which contains, as essential active components, Mo, V, P, Cs, $NH_4$ and Cu, the method including (a) a step of preparing a heteropoly acid aqueous solution or the like (A liquid) containing, as constituent elements, Mo, P and V; (b) a step of mixing a part of the resulting A liquid with a cesium compound aqueous solution or the like (B liquid); and (c) a step of mixing the remainder of the A liquid with the B liquid to prepare a slurry liquid (C liquid).

**Description**

Technical Filed

The present invention relates to a catalyst capable of stably producing a catalyst for producing methacrylic acid by subjecting methacrolein, isobutyl aldehyde or isobutyric acid to vapor phase catalytic oxidation without impairing a catalytic performance and a method for producing the same. The invention also relates to a method for producing methacrylic acid using the catalyst.

Background Art

A large number of catalysts have been proposed as a catalyst which is used for producing methacrylic acid by subjecting methacrolein, isobutyl aldehyde or isobutyric acid to vapor phase catalytic oxidation. These catalysts are those containing molybdenum and phosphorus as main components and having a structure of a heteropoly acid and/or a salt thereof. In addition, a large number of production methods of these catalysts have been similarly proposed.

For example, Patent Document 1 discloses a method for preparing a catalyst for synthesizing acrolein or methacrolein by mixing two or more kinds of solutions or dispersion liquids containing catalytic components within a short period of time as far as possible, spray drying the resulting mixture immediately thereafter without being aged, and then calcining the resulting dry product.

Patent Document 2 discloses that a solution or slurry (A liquid) containing molybdenum and phosphorus, a solution or slurry (B liquid) containing an alkali metal and/or an alkaline earth metal, and a solution or slurry (C liquid) containing an ammonium radical are used, in which the A liquid and the B liquid are mixed, and the C liquid is then mixed. However, Patent Document 2 discloses that it is preferable that the B liquid does not contain molybdenum or phosphorus.

Patent Document 3 discloses a method for preparing a heteropoly acid-based catalyst through two stages including obtaining a heteropoly acid containing at least molybdenum, phosphorus and cesium and then adding a catalyst raw material containing at least molybdenum and phosphorus but not containing cesium to the resulting heteropoly acid salt.

With respect to these known technologies, since Patent Document 1 relates to a preparation method in which not only mixing is performed in a short time, but immediately thereafter, spray drying is performed, there is a concern about the method of stably producing a catalyst. In Patent Document 2, the mixing method of a preparation liquid is clearly elucidated. However, in the case where the B liquid contains molybdenum or phosphorus, a more improvement is required in the yield of methacrylic acid. In Patent Document 3, the mixing method of a preparation liquid is clearly elucidated. However, after adding cesium, the mixture goes through a drying step, the heteropoly acid raw material is again added, followed by heating. Accordingly, this method is not economical. In addition, the catalysts obtained in the manners as in Patent Documents 1 to 3 are not satisfactory yet in the reaction results, and hence, it is the present situation that more improvements are desired on the occasion of use as an industrial catalyst.

Background Art Document

Patent Document

    [Patent Document 1] JP-A-H04-182449
    [Patent Document 2] JP-A-2007-283265
    [Patent Document 3] JP-A-H05-177141

Summary of Invention

Problem that Invention is to Solve

The invention is aimed to provide a catalyst for producing methacrylic acid in high yield and high selectivity by subjecting methacrolein, isobutyl aldehyde or isobutyric acid to vapor phase catalytic oxidation and a method for producing the same.

Means for Solving Problem

The present inventors have found that in a heteropoly acid neutralized salt compound containing, as essential active components, Mo, V, P, Cs, $NH_4$ and Cu, a catalyst produced by a specified method has an extremely high catalytic performance, leading to accomplishment of the invention.

Specifically, the invention is concerned with the following.

(1) A method for producing a catalyst for producing methacrylic acid having a composition represented by the following general formula (1):

$$Mo_{10}V_aP_b(NH_4)_cCs_dCu_eX_fO_g \qquad (1)$$

wherein Mo represents molybdenum; V represents vanadium; P represents phosphorus; ($NH_4$) represents an ammonium group; Cs represents cesium; Cu represents copper; X represents at least one element selected from the group consisting of Sb, As, Ag, Mg, Zn, Al, B, Ge, Sn, Pb, Ti, Zr, Cr, Re, Bi, W, Fe, Co, Ni, Ce and Th; O represents oxygen; a to g represent atomic ratios of the respective elements; a is satisfied with $(0.1 \leq a \leq 6.0)$; b is satisfied with $(0.5 \leq b \leq 6.0)$; c is satisfied with $(0.1 \leq c \leq 10.0)$; d is satisfied with $(0.1 \leq d \leq 3.0)$; e is satisfied with $(0.1 \leq e \leq 3)$; f is satisfied with $(0 \leq f \leq 3)$; and g is a numerical value determined according to oxidation states and atomic ratios of the respective elements other than O,

the method comprising the steps of:

(a) preparing a heteropoly acid aqueous solution or heteropoly acid aqueous dispersion (hereinafter referred to as "A liquid") containing, as constituent elements, molybdenum, phosphorus and vanadium;
(b) mixing a part of the A liquid obtained in the step (a) with an aqueous solution or aqueous dispersion containing a cesium compound to prepare a slurry liquid (hereinafter referred to as "B liquid");
(c) mixing the remainder of the A liquid with the B liquid to prepare a slurry liquid (hereinafter referred to as "C liquid");
(d) adding an ammonium compound to the C liquid obtained in the step (c) to obtain a slurry liquid;
(d') mixing an aqueous solution or aqueous dispersion containing copper on the way or after completion of the steps (a) to (d);
(e) drying the slurry liquid obtained in the step (d) or the step (d') after the step (d) to obtain a catalytically active component solid;
(f) molding the catalytically active component solid obtained in the step (e); and
(g) calcining a molded product obtained in the step (f).

(2) The method for producing a catalyst for producing methacrylic acid as described in (1) above, wherein in the step (b), an electric conductivity of the B liquid lies in a point neutralization.
(3) The method for producing a catalyst for producing methacrylic acid as described in (1) or (2) above, wherein in the step (b), a temperature of the aqueous solution or aqueous dispersion containing the A liquid and the cesium compound is from 0 to 35°C.
(4) The method for producing a catalyst for producing methacrylic acid as described in (1) above, further comprising the following step of:

(d") mixing an aqueous solution or aqueous dispersion containing X on the way or after completion of the steps (a) to (d) and the step (d').

(5) A catalyst for producing methacrylic acid, which is obtained by the method as described in any one of (1) to (4) above.
(6) A method for producing methacrylic acid, comprising:

partially oxidizing at least one compound selected from the group consisting of methacrolein, isobutyl aldehyde and isobutyric acid with the catalyst as described in (5) above in the presence of molecular oxygen.

Effects of Invention

According to the invention, it is possible to provide a catalyst containing, as essential active components, Mo, V, P, Cs, $NH_4$ and Cu in high yield and high selectivity and a method for producing the same.

Brief Description of the Drawing

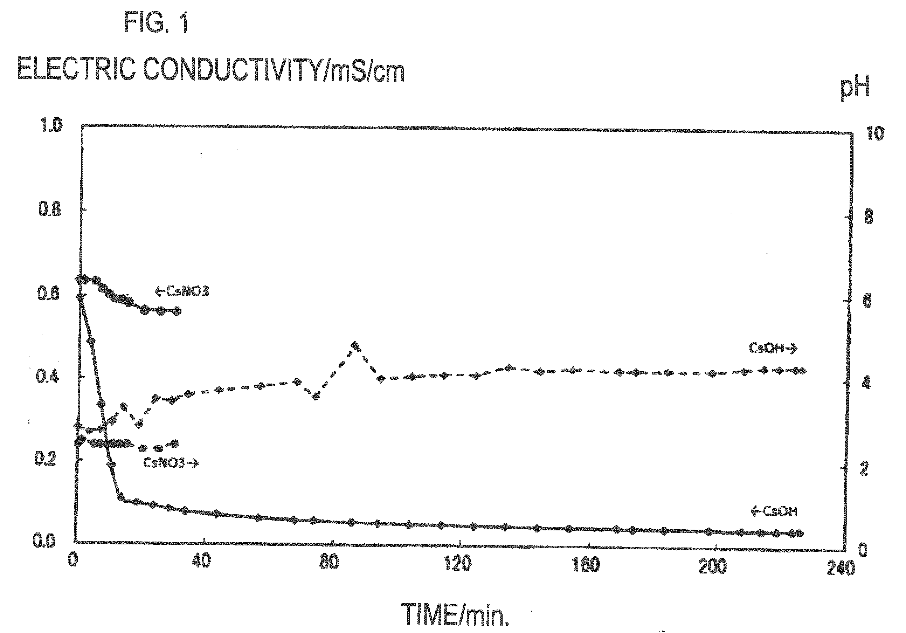

FIG. 1 is a graph in which an electric conductivity and a pH value of a mixed liquid in the step (b) of each of Examples 1 and 2 are shown on a Y axis, and a time is shown in terms of a minute unit on an X axis. A solid line expresses the electric conductivity, and a dotted line expresses the pH value.

Mode for Carrying Out Invention

The catalyst for producing methacrylic acid which can be produced by the production method of the invention is one to be used on the occasion of subjecting methacrolein to vapor phase catalytic oxidation with molecular oxygen to produce methacrylic acid and has a composition represented by the following general formula (1).

$$Mo_{10}V_aP_b(NH_4)_cCs_dCu_eX_fO_g \qquad (1)$$

In the foregoing formula (1), Mo represents molybdenum; V represents vanadium; P represents phosphorus; $(NH_4)$ represents an ammonium group; Cs represents cesium; Cu represents copper; X represents at least one element selected from the group consisting of Sb, As, Ag, Mg, Zn, Al, B, Ge, Sn, Pb, Ti, Zr, Cr, Re, Bi, W, Fe, Co, Ni, Ce and Th; O represents oxygen; a to g represent atomic ratios of the respective elements; a is satisfied with $(0.1 \leq a \leq 6.0)$; b is satisfied with $(0.5 \leq b \leq 6.0)$; c is satisfied with $(0.1 \leq c \leq 10.0)$; d is satisfied with $(0.1 \leq d \leq 3.0)$; e is satisfied with $(0.1 \leq e \leq 3)$; f is satisfied with $(0 \leq f \leq 3)$; and g is a numerical value determined according to the oxidation states and atomic ratios of the respective elements other than O.

In the foregoing general formula, the X component is preferably at least one element selected from the group consisting of Sb and As.

Preferred embodiments are hereunder described for every step as described above.

Step (a):

As for the active component-containing compound which is used for the preparation of a catalyst, when in addition to molybdenum, phosphorus and vanadium, each of which is an essential active component element in the step (a), essential active component elements in the step (d') and arbitrary active component elements in the step (d") are exemplified, examples thereof include chlorides, sulfates, nitrates, oxides and acetates of the active component elements. More specifically, preferred examples of the compound include nitrates such as cobalt nitrate; acetates such as copper acetate; oxides such as molybdenum oxide, vanadium pentoxide, copper oxide, antimony trioxide, cerium oxide, zinc oxide, and germanium oxide; and acids (or salts thereof) such as orthophosphoric acid, phosphoric acid, boric acid, aluminum phosphate, and 12-tungstophosphoric acid. The molybdenum oxide which is used for the production can be properly used in an average particle diameter ranging from 0.5 $\mu$m to 100 $\mu$m. These active component-containing compounds may be used solely or may be used in admixture of two or more kinds thereof. The slurry liquid can be obtained by uniformly mixing each active component-containing compound with water. The amount of water used in the slurry liquid is not particularly limited so long as the whole of the compound used can be completely dissolved, or the compound used can be uniformly mixed. Taking a drying method or a drying condition into consideration, the amount of water used may be properly determined. In general, the amount of water is from about 200 to 2,000 parts by mass based on 100 parts by mass of a total mass of the compound for the preparation of slurry. Though the amount of water may be large, when it is excessive, there is often brought such a disadvantage that the energy costs of the drying step become high, or drying may not be completely achieved.

As for a temperature on the occasion of preparing a slurry liquid, it is preferable to perform heating to a temperature at which the compound containing molybdenum, phosphorus and vanadium and optionally, other active component element can be thoroughly dissolved.

Step (b):

As for the cesium compound which is added, though any cesium compound may be used, cesium hydroxide or a weak acid salt such as cesium acetate and cesium carbonate is more preferable.

However, since it takes a long period of time until the electric conductivity is neutralized as described below, for the purpose of shortening the time, it is also possible to use a sulfate, nitrate or chloride of cesium, or add a pH modifier such as hydrogen peroxide water. This may be considered to be caused due to the matter that by keeping a low pH region, the heteropoly acid becomes stable, and a heteropoly acid neutralized salt is more quickly formed. The electric conductivity may be measured by either an AC two-electrode method or an electromagnetic induction method. However, since the slurry liquid in the vicinity of a point of neutralization frequently becomes low in conductivity, it is preferable to measure the electric conductivity by an AC two-electrode method. Incidentally, though a conductivity meter (CM-60G), manufactured by DKK-TOA Corporation is preferable as a measuring instrument, it should not be construed that the measuring instrument is limited thereto.

In the step (b), a part of the A liquid is mixed with an aqueous solution or aqueous dispersion of the cesium compound.

Though the part of the A liquid in the step (b) is determined by the atomic ratio d of the cesium to be added, it is added in an amount accounting for from 5 to 20% by weight, and preferably from 10 to 15% by weight in terms of a liquid amount relative to the whole of the A liquid.

In the step (b), it is preferable to perform mixing in a ratio such that the electric conductivity of the mixture (B liquid) of a part of the A liquid and an aqueous solution or aqueous dispersion of the cesium compound lies in a point neutralization. For this reason, it is preferable that the amount of the heteropoly acid which is contained in the A liquid falls within the range that is a stoichiometric amount in the heterocyclic acid relative to the atomic ratio d of cesium.

Different from the invention, in the case of adding the cesium compound to the whole of the A liquid, there is a concern that the selectivity of methacrylic acid is lowered. This may be considered to be caused due to the matter that the cesium compound forms a partially neutralized salt of the heteropoly acid in a state where the cesium compound is highly dispersed, and the acid strength of the catalyst becomes strong, whereby decomposition of methacrylic acid is promoted.

Step (c):

Subsequently, the remainder of the A liquid is mixed with the B liquid obtained in the step (b) to obtain a slurry liquid (C liquid). Incidentally, in the steps (b) and (c), a temperature of the A liquid is generally in the range of from about 0 to 35°C, and preferably from about 0 to 30°C. In that case, the resulting catalyst tends to become high in activity.

Step (d):

Subsequently, the C liquid obtained in the step (c) is mixed with an ammonium compound to obtain a slurry liquid. The ammonium compound which is used in the step (d) is preferably ammonium acetate or ammonium hydroxide.

Steps (d') and (d"):

In the case where a copper component is added, and an X component is added as the need arises, the addition step is not particularly limited, and the component or components may be properly added on the way or after completion of the steps (a) to (d).

In the invention, the shape of a stirring blade of a stirrer which is used on the occasion of adding the essential active components is not particularly limited, and an arbitrary stirring blade such as a propeller blade, a turbine blade, a paddle blade, a pitched paddle blade, a screw blade, an anchor blade, a ribbon blade, and a large-sized lattice blade can be used in a single stage or two or more stages of the same blade or a combination of different kinds of blades in the vertical direction. In addition, a baffle (turning blade) may be installed in a reaction vessel as the need arises.

Step (e):

The slurry liquid which has gone through the step (d) or the step (d') after the step (d) is dried to obtain a catalytically active component solid. Though the drying method in the step (e) is not particularly limited so long as the slurry liquid can be completely dried, examples thereof include drum drying, freeze drying, spray drying, and evaporation to dryness. Of these, spray drying is preferable in the invention because the slurry liquid can be dried into a powder or granule within a short period of time. Though a drying temperature of the spray drying varies depending upon the concentration or liquid feed rate of the slurry liquid, or the like, it is approximately from 70 to 150°C in terms of a temperature at an outlet of a drying machine. In addition, it is preferable to perform the drying such that an average particle diameter of the catalytically active component solid obtained on this occasion is from 10 to 700 $\mu$m.

Step (f):

The catalytically active component solid obtained in the above-described step (e) is molded.

The molding method in the step (f) is not particularly limited. In the case of using the catalytically active component solid for oxidation reaction, in order to decrease a pressure loss of the reaction gas, the catalytically active component solid is molded in a columnar, tablet-like, ring-like or spherical form, or the like. Above all, it is preferable to coat the catalytically active component solid on an inert carrier to form a coated catalyst because an enhancement of the selectivity or removal of heat of reaction can be expected. A rolling granulation method as described below is preferable for this coating step. This method is, for example, a method in which in an apparatus having a flat or concave and convex disc in the bottom of a fixed container, the disc is rotated at a high speed, thereby vigorously agitating the carrier in the container by repeating rotation movement and revolution movement, and a binder and a coating mixture prepared by adding the catalytically active component solid and optionally, other additive such as a molding assistance and a strength improvement are coated on the carrier. As an addition method of the binder, a method such as (1) a method of previously mixing the binder with the coating mixture; (2) a method of adding the binder simultaneously with the addition of the coating mixture in the fixed container; (3) a method of adding the coating mixture in the fixed container and then adding the binder; (4) a method of adding the binder before adding the coating mixture in the fixed container; and (5) a method of dividing each of the coating mixture and the binder and adding the whole by properly combining the methods (2) to (4) can be adopted. Above all, in the method (5), for example, it is preferable to add the binder while regulating the addition rate using an auto feeder or the like such that a prescribed amount of the catalytically active component solid is carried on the carrier without causing attachment of the coating mixture onto a wall of the fixed container or coating mixture-to-coating mixture coagulation. The binder is preferably water, or at least one member selected from the group consisting of organic compounds having a boiling point of not higher than 150°C at one atmosphere or lower, or an aqueous solution thereof. Specific examples of the binder other than water include alcohols such as methanol, ethanol, propanols, and butanols, and preferably alcohols having from 1 to 4 carbon atoms; ethers such as ethyl ether, butyl ether, and dioxane; esters such as ethyl acetate and butyl acetate; ketones such as acetone and methyl ethyl ketone; and aqueous solutions thereof. In particular, ethanol is preferable. In the case of using ethanol as the binder, a ratio of ethanol to water is from 9.9/0.1 to 0.1/9.9 (mass ratio), and it is preferable to mix ethanol with water in a ratio of 9/1 to 1/9 (mass ratio). The amount of such a binder used is generally from 2 to 60 parts by mass, and preferably from 10 to 50 parts by mass based on 100 parts by mass of the coating mixture. When the catalytic active component solid is calcined at from about 250°C to 350°C, followed by molding, there may be the case where the mechanical strength or catalytic performance is enhanced, and such is preferable.

Specific examples of the carrier in the above-described coating include spherical carries having a diameter of from 1 to 15 mm, and preferably from 2.5 to 10 mm, such as silicon carbide, alumina, silica alumina, mullite, and Alundum. As such a carrier, one having a porosity of from 10 to 70% is generally used. As for a proportion of the carrier and the coating mixture, the carrier is used such that a ratio of the coating mixture to the total of the coating mixture and the carrier is generally from 10 to 75% by mass, and preferably from 15 to 60% by mass. In the case where the proportion of the coating mixture is too large, though the reactive activity of the coated catalyst is large, the mechanical strength tends to become small. Conversely, in the case where the proportion of the coating mixture is too small, though the mechanical strength is large, the reactive activity tends to become small. Incidentally, in the foregoing, examples of the molding assistant which is used as the need arises include a silica gel, diatomaceous earth, and an alumina powder. The amount of the molding assistant used is generally from 1 to 60 parts by mass based on 100 parts by mass of the catalytically active component solid. In addition, what an inorganic fiber (for example, a ceramic fiber, a whisker, etc.) which is inert against the catalytically active component and the reactive gas is further added as a strength improver as the need arises is useful for enhancing the mechanical strength of the catalyst. In particular, a glass fiber is preferable. The amount of such a fiber used is generally from 1 to 30 parts by mass based on 100 parts by mass of the catalytically active component solid.

Step (g):

The coated catalyst obtained in the step (f) can be directly subjected as a catalyst to a catalytic vapor phase oxidation reaction. However, there may be the case where when calcined, the catalytic activity of the coated catalyst is enhanced, and therefore, it is preferable to calcine the coated catalyst. A calcination temperature is generally from 100°C to 450°C, preferably from 250°C to 420°C, more preferably 250°C or higher and lower than 400°C, and still more preferably 300°C or higher and lower than 400°C. A calcination time is from 1 to 20 hours. Incidentally, though the calcination is generally performed in an air atmosphere, it may also be performed in an inert gas atmosphere of nitrogen, etc., or in a reductive gas atmosphere of ethanol, etc. After the calcination in an inert gas or reductive gas atmosphere, the calcination may be further performed in an air atmosphere. A proportion of the active component to the whole of the thus obtained coated catalyst is from 10 to 60% by mass.

The thus obtained catalyst (hereinafter referred to as "catalyst of the invention") is used for the production of methacrylic acid by means of vapor phase catalytic oxidation of methacrolein, isobutyl aldehyde or isobutyric acid. The vapor phase catalytic reaction with methacrolein that is the most preferable raw material for the use of the catalyst of the invention is hereunder described. Molecular oxygen or a molecular oxygen-containing gas is used for the vapor phase catalytic oxidation reaction. A proportion of the molecular oxygen used to methacrolein is preferably in the range of from 0.5 to 20, and especially preferably in the range of from 1 to 10 in terms of a molar ratio. For the purpose of making the reaction proceed smoothly, it is preferable to add water in an amount ranging from 1 to 20 in terms of a molar ratio to methacrolein in water. The raw material gas may contain, in addition oxygen and optionally, water (generally contained as water vapor), a gas which is inert against the reaction, such as nitrogen, carbon dioxide, and a saturated hydrocarbon. In addition, as the methacrolein, a gas obtained by oxidizing isobutylene, tertiary butanol, and methyl tertiary butyl ether may be fed as it is. A reaction temperature in the vapor phase catalytic oxidation reaction is generally from 200 to 400°C, and from 260 to 360°C, and the amount of the raw material gas fed is generally from 100 to 6,000 hr$^{-1}$, and preferably from 300 to 3,000 hr$^{-1}$ in terms of a space velocity. In addition, though it is possible to perform the vapor phase catalytic oxidation reaction either under elevated pressure or under reduced pressure, a pressure close to atmospheric pressure is generally suitable.

Examples

The invention is hereunder described in more detail by reference to the following Examples and Comparative Example, but it should not be construed that the invention is limited thereto.

Incidentally, in the following, conversion, selectivity, and yield are defined as follows.

$$\text{Conversion} = (\text{Molar number of methacrolein reacted})/(\text{Molar number of methacrolein fed}) \times 100$$

$$\text{Selectivity} = (\text{Molar number of methacrylic acid formed})/(\text{Molar number of methacrolein reacted}) \times 100$$

$$\text{Yield} = (\text{Molar number of methacrylic acid formed})/(\text{Molar number of methacrolein fed}) \times 100$$

Example 1

(1) Preparation of catalyst:

To 5,680 mL of pure water, 800 g of molybdenum trioxide, 30.33 g of vanadium pentoxide, and 76.87 g of 85% by mass of orthophosphoric acid, and the contents were heated and stirred at 92°C for 3 hours, thereby obtaining a reddish brown transparent solution (A liquid). An average particle diameter of the molybdenum trioxide used at that time was 1.5 $\mu$m. Subsequently, this solution was cooled to 0 to 20°C, 944.31 g of which was then collected in another container. Thereafter, to this solution collected in another container, 661.32 g of a 9.1% by mass cesium hydroxide aqueous solution was gradually added while stirring, and the contents were aged at 15 to 20°C until its electric conductivity was neutralized, thereby obtaining a yellow slurry liquid (B liquid). The electric conductivity was measured using a conductivity meter (CM-60G), manufactured by DKK-TOA Corporation. Incidentally, changes of the electric conductivity and pH value (Y axis) with time (X axis) of the mixed liquid obtained by adding the cesium hydroxide aqueous solution to the A liquid are shown in FIG. 1. Subsequently, the B liquid was added to the remainder of the A liquid, to which was then gradually added 196.86 g of a 50.0% by mass ammonium acetate aqueous solution while stirring, and the contents were aged at 0°C to 30°C for one hour. Subsequently, 22.18 g of cupric acetate was further added to the resulting slurry, and the contents were stirred and mixed at 0 to 30°C until they were completely dissolved. Subsequently, this slurry was spray dried to obtain a catalytically active component solid. A composition of the catalytically active component solid as determined from the amounts of the raw materials charged is as follows.

$$Mo_{10}V_{0.6}P_{1.1}Cs_{0.7}(NH_4)_{2.3}Cu_{0.3}$$

Subsequently, 120 g of a complex oxide and 6.5 g of a strength improver (glass fiber) were uniformly mixed, and the mixture was subjected to coating molding on 200 g of a spherical porous alumina carrier (particle diameter: 4.5 mm) with about 30 g of a 50% by mass ethanol aqueous solution as a binder. Subsequently, the resulting molded product was calcined under ventilation with air at 380°C over 5 hours, thereby obtaining a desired coated catalyst. The ammonia component that is an active component composition after the calcination became about 0.01 to 1.0. This may be considered to have been caused due to the matter that the ammonia component was lost by the calcination.

(2) Catalytic oxidation reaction of methacrolein:

10.3 mL of the resulting coated catalyst was filled in a stainless steel reaction tube having an inner diameter of 18.4 mm, and a raw material gas (composition (molar ratio); methacrolein/oxygen/water vapor/nitrogen = 1/2/4/18.6) was subjected to oxidation reaction of methacrolein under a condition at a space velocity (SV) of 1,200 hr$^{-1}$. The reaction was performed by raising a reaction bath temperature to 350°C and continued for 15 hours. Subsequently, the reaction bath temperature was decreased to 310°C, and the reaction results were measured.

Example 2

A coated catalyst was prepared by the same method as that in Example 1, except that in Example 1, 661.32 g of the 9.1% by mass cesium hydroxide aqueous solution was changed to 417.07 g of a 9.1% by mass cesium nitrate aqueous solution, and then subjected to the catalytic oxidation reaction of methacrolein. Incidentally, changes of the electric conductivity and pH value (Y axis) with time (X axis) of the mixed liquid obtained by adding the cesium nitrate aqueous solution to the A liquid are shown in FIG. 1.

Example 3

A coated catalyst was prepared by the same method as that in Example 1, except that in Example 1, 15.51 g of 30% by mass hydrogen peroxide water was added before adding cesium hydroxide, and then subjected to the catalytic oxidation reaction of methacrolein.

Example 4

In Example 1, after putting the B liquid, 13.15 g of 60% by mass arsenic acid was gradually added, and the contents were aged at 0°C to 30°C for one hour. Then, 196.86 g of a 50.0% by mass ammonium acetate aqueous solution was gradually added. Thereafter, a coated catalyst was prepared by the same method as that in Example 1 and then subjected to the catalytic oxidation reaction of methacrolein.

Example 5

A coated catalyst was prepared by the same method as that in Example 1, except that in Example 1, after adding Cs, an ammonium acetate aqueous solution was added in a state where the electric conductivity was not neutralized, specifically immediately after completion of the addition of the cesium hydroxide aqueous solution, and then subjected to the catalytic oxidation reaction of methacrolein.

Example 6

A coated catalyst was prepared by the same method as that in Example 1, except that in Example 1, after adding Cs, an ammonium acetate aqueous solution was added in a state where the electric conductivity was not neutralized, specifically 0.5 hours after completion of the addition of the cesium hydroxide aqueous solution, and then subjected to the catalytic oxidation reaction of methacrolein.

Comparative Example 1

To 5,680 mL of pure water, 800 g of molybdenum trioxide, 30.33 g of vanadium pentoxide, and 76.87 g of 85% by mass orthophosphoric acid were added, and the contents were heated and stirred at 92°C for 3 hours, thereby obtaining a reddish brown transparent solution. Subsequently, after cooling this solution to 0 to 20°C, 661.32 g of a 9.1% by mass cesium hydroxide aqueous solution was gradually added while stirring, and the contents were aged for one hour to obtain a yellow slurry liquid. Subsequently, to the resulting slurry liquid, 196.86 g of a 50.0% by mass ammonium acetate aqueous solution was gradually added while stirring, and the contents were aged at 0°C to 30°C for one hour. Subsequently, 22.18 g of cupric acetate was further added to the slurry, and the contents were stirred and mixed at 0 to 30°C until they were completely dissolved. Subsequently, this slurry was spray dried to obtain a catalytically active component solid. Subsequently, a coated catalyst was prepared by the same method as that in Example 1 and then subjected to the catalytic oxidation reaction of methacrolein.

Table 1

| | Time from adding the cesium raw material to a part of the A liquid until adding the remainder of the A liquid [hr] | Electric conductivity of the B liquid on the occasion of adding the remainder of the A liquid [mS/cm] | Conversion of methacrolein [%] | Selectivity of methacrylic acid [%] | Yield of methacrylic acid [%] |
|---|---|---|---|---|---|
| Example 1 | 3.7 | 0.042 | 89.60 | 84.84 | 76.02 |
| Example 2 | 0.5 | 0.562 | 83.66 | 87.50 | 73.20 |
| Example 3 | 2.2 | 0.653 | 87.60 | 86.34 | 75.63 |
| Example 4 | 4.0 | 0.071 | 85.51 | 87.91 | 75.17 |
| Example 5 | 0 | 0.817 | 76.80 | 86.22 | 66.21 |
| Example 6 | 0.5 | 0.527 | 83.20 | 86.33 | 71.83 |
| Comparative Example 1 | 1.0 | 0.427 | 88.47 | 84.46 | 74.72 |

While the invention has been described in detail and with reference to specific embodiments thereof, it will be apparent to one skilled in the art that various changes and modifications can be made therein without departing from the spirit and scope thereof.

Incidentally, the present application is based on a Japanese patent application filed on March 28, 2013 (Japanese Patent Application No. 2013-069908), the entirety of which is incorporated by reference. In addition, all references cited herein are incorporated as a whole.

Industrial Applicability

According to the production method of the invention, a catalyst for producing methacrylic acid in high yield and high selectivity by subjecting methacrolein, isobutyl aldehyde or isobutyric acid to vapor phase catalytic oxidation and a method for producing the same can be provided.

**Claims**

1. A method for producing a catalyst for producing methacrylic acid having a composition represented by the following general formula (1):

$$Mo_{10}V_aP_b(NH_4)_cCs_dCu_eX_fO_g \qquad (1)$$

wherein Mo represents molybdenum; V represents vanadium; P represents phosphorus; (NH$_4$) represents an ammonium group; Cs represents cesium; Cu represents copper; X represents at least one element selected from the group consisting of Sb, As, Ag, Mg, Zn, Al, B, Ge, Sn, Pb, Ti, Zr, Cr, Re, Bi, W, Fe, Co, Ni, Ce and Th; O represents oxygen; a to g represent atomic ratios of the respective elements; a is satisfied with ($0.1 \leq a \leq 6.0$); b is satisfied with ($0.5 \leq b \leq 6.0$); c is satisfied with ($0.1 \leq c \leq 10.0$); d is satisfied with ($0.1 \leq d \leq 3.0$); e is satisfied with ($0.1 \leq e$ 3); f is satisfied with ($0 \leq f \leq 3$); and g is a numerical value determined according to oxidation states and atomic ratios of the respective elements other than O,
the method comprising the steps of:

(a) preparing a heteropoly acid aqueous solution or heteropoly acid aqueous dispersion (hereinafter referred to as "A liquid") containing, as constituent elements, molybdenum, phosphorus and vanadium;
(b) mixing a part of the A liquid obtained in the step (a) with an aqueous solution or aqueous dispersion containing a cesium compound to prepare a slurry liquid (hereinafter referred to as "B liquid");
(c) mixing the remainder of the A liquid with the B liquid to prepare a slurry liquid (hereinafter referred to as "C liquid");
(d) adding an ammonium compound to the C liquid obtained in the step (c) to obtain a slurry liquid;
(d') mixing an aqueous solution or aqueous dispersion containing copper on the way or after completion of the steps (a) to (d);

(e) drying the slurry liquid obtained in the step (d) or the step (d') after the step (d) to obtain a catalytically active component solid;
(f) molding the catalytically active component solid obtained in the step (e); and
(g) calcining a molded product obtained in the step (f).

2. The method for producing a catalyst for producing methacrylic acid according to claim 1,
   wherein in the step (b), an electric conductivity of the B liquid lies in a point neutralization.

3. The method for producing a catalyst for producing methacrylic acid according to claim 1 or 2,
   wherein in the step (b), a temperature of the aqueous solution or aqueous dispersion containing the A liquid and the cesium compound is from 0 to 35°C.

4. The method for producing a catalyst for producing methacrylic acid according to claim 1, further comprising the following step of:

   (d") mixing an aqueous solution or aqueous dispersion containing X on the way or after completion of the steps (a) to (d) and the step (d').

5. A catalyst for producing methacrylic acid, which is obtained by the method according to any one of claims 1 to 4.

6. A method for producing methacrylic acid, comprising:

   partially oxidizing at least one compound selected from the group consisting of methacrolein, isobutyl aldehyde and isobutyric acid with the catalyst according to claim 5 in the presence of molecular oxygen.

EP 2 979 757 A1

## FIG. 1

ELECTRIC CONDUCTIVITY/mS/cm

## INTERNATIONAL SEARCH REPORT

| International application No. |
| --- |
| PCT/JP2014/057990 |

**A. CLASSIFICATION OF SUBJECT MATTER**
*B01J27/199*(2006.01)i, *B01J37/00*(2006.01)i, *B01J37/04*(2006.01)i, *B01J37/08* (2006.01)i, *C07C51/235*(2006.01)i, *C07C57/055*(2006.01)i, *C07B61/00* (2006.01)n

According to International Patent Classification (IPC) or to both national classification and IPC

**B. FIELDS SEARCHED**

Minimum documentation searched (classification system followed by classification symbols)
B01J21/00-38/74, C07C1/00-409/44, C07B61/00

Documentation searched other than minimum documentation to the extent that such documents are included in the fields searched

| | | | |
| --- | --- | --- | --- |
| Jitsuyo Shinan Koho | 1922–1996 | Jitsuyo Shinan Toroku Koho | 1996–2014 |
| Kokai Jitsuyo Shinan Koho | 1971–2014 | Toroku Jitsuyo Shinan Koho | 1994–2014 |

Electronic data base consulted during the international search (name of data base and, where practicable, search terms used)

**C. DOCUMENTS CONSIDERED TO BE RELEVANT**

| Category* | Citation of document, with indication, where appropriate, of the relevant passages | Relevant to claim No. |
| --- | --- | --- |
| X | JP 2011-152543 A (Nippon Kayaku Co., Ltd.), 11 August 2011 (11.08.2011), claims 1 to 3, 5; paragraphs [0001], [0009] to [0021]; examples 1 to 8 (Family: none) | 1-6 |
| X | JP 2011-092882 A (Sumitomo Chemical Co., Ltd.), 12 May 2011 (12.05.2011), claims 1, 4, 6 to 8; paragraphs [0006], [0009], [0010], [0033] to [0037]; examples 1 to 5 & US 2011/0105789 A1 & CN 102049272 A | 1-6 |
| X | JP 09-024277 A (Sumitomo Chemical Co., Ltd.), 28 January 1997 (28.01.1997), claims 1, 2; paragraphs [0001], [0007], [0008], [0013], [0014], [0016], [0017]; examples 1 to 6 (Family: none) | 1-6 |

☒ Further documents are listed in the continuation of Box C. ☐ See patent family annex.

| | |
| --- | --- |
| * Special categories of cited documents:<br>"A" document defining the general state of the art which is not considered to be of particular relevance<br>"E" earlier application or patent but published on or after the international filing date<br>"L" document which may throw doubts on priority claim(s) or which is cited to establish the publication date of another citation or other special reason (as specified)<br>"O" document referring to an oral disclosure, use, exhibition or other means<br>"P" document published prior to the international filing date but later than the priority date claimed | "T" later document published after the international filing date or priority date and not in conflict with the application but cited to understand the principle or theory underlying the invention<br>"X" document of particular relevance; the claimed invention cannot be considered novel or cannot be considered to involve an inventive step when the document is taken alone<br>"Y" document of particular relevance; the claimed invention cannot be considered to involve an inventive step when the document is combined with one or more other such documents, such combination being obvious to a person skilled in the art<br>"&" document member of the same patent family |

| Date of the actual completion of the international search<br> 02 June, 2014 (02.06.14) | Date of mailing of the international search report<br> 24 June, 2014 (24.06.14) |
| --- | --- |
| Name and mailing address of the ISA/<br> Japanese Patent Office | Authorized officer |
| Facsimile No. | Telephone No. |

Form PCT/ISA/210 (second sheet) (July 2009)

**INTERNATIONAL SEARCH REPORT**

International application No.

PCT/JP2014/057990

C (Continuation). DOCUMENTS CONSIDERED TO BE RELEVANT

| Category* | Citation of document, with indication, where appropriate, of the relevant passages | Relevant to claim No. |
|---|---|---|
| X | JP 2003-190798 A (Mitsubishi Rayon Co., Ltd.), 08 July 2003 (08.07.2003), claims 1, 6, 7; paragraphs [0001], [0005], [0057], [0086]; examples 1 to 5 (Family: none) | 1-6 |

Form PCT/ISA/210 (continuation of second sheet) (July 2009)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP H04182449 A
- JP 2007283265 A
- JP H05177141 A
- JP 2013069908 A